# EUROPÄISCHE PATENTSCHRIFT

(11) **EP 3 559 659 B1**
(45) Veröffentlichungstag und Bekanntmachung des Hinweises auf die Patenterteilung: **31.07.2024**
(21) Anmeldenummer: 17816396.0
(22) Anmeldetag: 12.12.2017
(51) Int. Cl.: G01N 33/00, G01N 27/12

(54) **SENSORANORDNUNG ZUR BESTIMMUNG UND GEGEBENENFALLS MESSUNG EINER KONZENTRATION VON MEHREREN GASEN UND VERFAHREN ZUR HERSTELLUNG EINER SENSORANORDNUNG**
SENSOR ARRANGEMENT FOR DETERMINING AND POSSIBLY MEASURING A CONCENTRATION OF A PLURALITY OF GASES, AND METHOD FOR PRODUCING A SENSOR ARRANGEMENT
DISPOSITIF DE DÉTECTION POUR DÉTERMINER ET, LE CAS ÉCHÉANT, MESURER UNE CONCENTRATION DE PLUSIEURS GAZ ET PROCÉDÉ POUR FABRIQUER UN DISPOSITIF DE DÉTECTION

(30) Priorität: 22.12.2016 AT 511742016
(43) Veröffentlichungstag der Anmeldung: 30.10.2019
(73) Patentinhaber: Materials Center Leoben Forschung GmbH, 8700 Leoben (AT)
(72) Erfinder: WIMMER-TEUBENBACHER, Robert, 8010 Graz (AT); KÖCK, Anton, 1130 Wien (AT); DEFREGGER, Stefan, 8501 Lieboch (AT)
(74) Vertreter: Wirnsberger, Gernot
(86) Internationale Anmeldenummer: PCT/AT2017/060330
(87) Internationale Veröffentlichungsnummer: WO 2018/112486

(56) Entgegenhaltungen:
- DE-C1- 19 718 584
- GB-A- 2 527 340
- JP-A- H07 294 470
- US-A- 5 783 154
- US-A1- 2011 120 866
- US-A1- 2016 223 490

## Beschreibung

Die Erfindung betrifft eine Sensoranordnung zur Bestimmung und gegebenenfalls Messung einer Konzentration von mehreren Gasen, umfassend eine Sensorstruktur mit in einer Matrix angeordneten leitenden Elementen und einer Vielzahl von Kontakten, wobei die leitenden Elemente mit den Kontakten verbunden sind.

Des Weiteren betrifft die Erfindung eine Verwendung einer solchen Sensoranordnung.

Weiter betrifft die Erfindung ein Verfahren zur Herstellung einer Sensoranordnung zur Bestimmung und gegebenenfalls Messung einer Konzentration von mehreren Gasen.

Gassensoren sind aus dem Stand der Technik bekannt und zur Detektion von gasförmigen Substanzen ausgebildet. Insbesondere werden zur Messung von Gasen Metalloxidsensoren verwendet. Diese werden beispielsweise zur Detektion von Kohlenmonoxid eingesetzt. Nachteilig bei solchen aus dem Stand der Technik bekannten Sensoren ist allerdings, dass diese entweder auf mehrere Gase reagieren bzw. nicht zwischen zwei oder mehr Gasarten unterscheiden können.

In der US 2011/0120866 A1 ist ein Gassensor offenbart, welcher dazu ausgebildet ist, gleichzeitig zwei Gase zu detektieren. Auch mit einem solchen Gassensor ist es jedoch nicht möglich, mehr als zwei Gasarten isoliert voneinander zu messen.

Die EP 0 527 258 A1 offenbart ein Gassensorarray zum Detektieren von einzelnen Gaskomponenten in einem Gasgemisch. Ein solches Gassensorarray ist jedoch nicht derart flexibel konfigurierbar, dass zwei oder mehr Gase gleichzeitig bestimmbar sind, da eine Bestimmung eines Gases erst durch Berechnungen aus Sensorsignalen möglich ist.

Hier setzt die Erfindung an. Aufgabe der Erfindung ist es, eine Sensoranordnung der eingangs genannten Art anzugeben, welche flexibel konfigurierbar ist.

Ein weiteres Ziel ist es, eine Verwendung einer solchen Sensoranordnung anzugeben.

Weiter ist es ein Ziel, ein Verfahren der eingangs genannten Art anzugeben, mit welchem eine flexibel konfigurierbare Sensoranordnung hergestellt werden kann.

Die Aufgabe wird erfindungsgemäß dadurch gelöst, dass bei einer Sensoranordnung der eingangs genannten Art die leitenden Elemente mit unterschiedlichen Metalloxiden gebildet sind, um in einem Gasgemisch mehr als zwei Gase isoliert voneinander zu messen.

Ein mit der Erfindung erzielter Vorteil ist insbesondere darin zu sehen, dass durch die Ausbildung der Sensorstruktur, insbesondere der in einer Matrix angeordneten bzw. als Matrix ausgebildeten leitenden Elemente eine flexibel konfigurierbare Sensoranordnung geschaffen ist, mit welcher mehr als zwei Gase eines Gasgemisches erkennbar bzw. messbar sind. Die leitenden Elemente sind zumindest teilweise aus zumindest zwei unterschiedlichen Metalloxiden gebildet. Bei einer erfindungsgemäßen Sensoranordnung sind die einzelnen leitenden Elemente aus unterschiedlichen Metalloxiden in der Matrix derart angeordnet bzw. miteinander verschaltet, dass diese beliebig extern über die Kontakte miteinander koppelbar und gegeneinander verschaltbar sind, um mehr als zwei unterschiedliche Gase individuell zu bestimmen und darüber hinaus gegebenenfalls eine Konzentration der einzelnen Gase zu messen. Jeder Kontakt ist zumindest mit einem leitenden Element verbunden, um in weiterer Folge ein Vorhandensein eines Gases feststellen zu können. Durch die Ausbildung der Sensoranordnung aus unterschiedlichen Metalloxiden bildet die Sensoranordnung mehrere miteinander koppelbare Sensoren aus, welche individuell über die Kontakte ansteuerbar und untereinander verschaltbar sind. Die Sensoren sind insbesondere an Berührungspunkten zwischen zwei oder mehr leitenden Elementen angeordnet. Darüber hinaus sind die Sensoren beabstandet voneinander angeordnet.

Insbesondere sind die Sensorstruktur bzw. die leitenden Elemente aus oder mit zwei verschiedenen Metalloxiden gebildet, bevorzugt aus Kupferoxid und/oder Zinkoxid und/oder Zinnoxid. Die Sensorstruktur umfasst beispielsweise eine Matrix aus gitterförmig angeordneten Strängen aus unterschiedlichen Metalloxiden. Hierbei ist es günstig, wenn die Stränge etwa senkrecht zueinander angeordnet und aus unterschiedlichen Metalloxiden ausgebildet sind. Die Stränge laufen an Kreuzungspunkten übereinander und können an diesen

Kreuzungspunkten miteinander verbunden sein, wobei die Kreuzungspunkte beabstandet zueinander angeordnet sind. Eine solche vertikale Anordnung zweier unterschiedlicher Metalloxide kann beispielsweise einen p-n Übergang bilden. Mit Vorteil können die Stränge auch aus Schichten und/oder Nanofasern gebildet sein. Darüber hinaus können auch unterschiedliche Kontaktelemente matrixförmig auf einem Substrat angeordnet sein, wobei diese auch teilweise oder vollständig in unterschiedliche Metalloxide umgewandelt werden können und gitterförmig miteinander verbunden sind.

Es ist günstig, wenn die Matrix Metalloxidfilme umfasst. Die Metalloxidfilme sind insbesondere streifenförmig ausgebildet und gitterförmig bzw. matrixförmig auf einem Substrat angeordnet. An jeweiligen Enden der streifenförmigen Metalloxidfilme sind Kontakte angeordnet, über welche unterschiedlich ausgebildete sowie beabstandet voneinander angeordnete Sensoren ansteuerbar sind. Sensoren sind an oder zwischen Kreuzungspunkten der Metalloxidfilme ausgebildet, wobei die Kreuzungspunkte beabstandet voneinander angeordnet sind. Insbesondere sind Metallfilme in weiterer Folge zu Metalloxidfilmen und/oder Metalloxidnanofasern oxidierbar, wodurch aus Metallfilmen gassensitive Metalloxide werden. Es ist dabei zweckmäßig, wenn jede Reihe bzw. Spalte der Metallfilme in ein unterschiedliches Metalloxid umwandelbar ist, beispielsweise durch thermische Oxidation. Es kann weiter vorgesehen sein, dass die Metalloxidfilme als Bündel von Nanofasern ausgebildet sind.

Vorteilhaft ist es, wenn die Sensorstruktur Nanofasern umfasst. Die Nanofasern sind hierbei auf bzw. zwischen leitenden Elementen der Metallmatrix angeordnet, wobei die Nanofasern über einen Transferprozess auf ein Substrat übertragen werden, beispielsweise thermisch darauf oxidiert. Die Nanofasern sind bevorzugt aus Metalloxiden sowie gassensitiv, beispielsweise durch deren elektrische Leitfähigkeit, ausgebildet, wodurch eine Empfindlichkeit der Sensoranordnung weiter erhöht ist. Die Nanofasern können auch über Oxidation von geeigneten Metallfilmen auf einem Metallgitter hergestellt sein.

Besonders bevorzugt ist es, wenn Kontaktelemente, insbesondere metallische und/oder metalloxidische Kontaktelemente vorgesehen sind, wobei die Nanofasern zwischen den Kontaktelementen angeordnet sind. Mit Vorteil sind jeweils zwei Nanofasern so zueinander orientiert, dass die zweite Nanofaser in einem Winkel zwischen 45° und 135°, besonders bevorzugt etwa senkrecht zur ersten Nanofaser ausgerichtet ist. Die mit den Nanofasern verbundenen Kontaktelemente bilden dadurch ein Gitter aus, wobei bevorzugt Bündel von Nanofasern vorgesehen sind. Die Kontaktelemente sind mit Vorteil etwa rechteckig, insbesondere quadratisch, ausgebildet und matrixförmig auf einem Substrat angeordnet. Die äußeren Kontaktelemente der dadurch gebildeten Matrix sind jeweils mit einem Kontakt verbunden. Die Kontaktelemente können direkt auf einem Substrat wachsen oder auf einem solchen aufgedampft werden. Es kann vorgesehen sein, dass die Kontaktelemente und/oder die Nanofasern aus unterschiedlichen Metallen und/oder Metalloxiden ausgebildet sind.

Es ist günstig, wenn die Nanofasern dotiert sind. Es werden Fremdatome in die jeweiligen Nanofasern aus einem Metall und/oder Metalloxid eingebracht, um diese für unterschiedliche Gase empfindlich bzw. unterschiedlich empfindlich zu machen.

Es ist weiter zweckmäßig, wenn die Sensorstruktur Nanopartikel umfasst, um Sensoren unterschiedlich zu sensibilisieren. Die Sensorstruktur ist dadurch unterschiedlich funktionalisierbar. Durch die Nanopartikel sind den in der Sensorstruktur ausgebildeten Sensoren unterschiedliche Eigenschaften und/oder Sensibilitäten zuordenbar, wodurch in weiterer Folge in einem Gasgemisch mehr als zwei Gase isoliert voneinander bzw. einzeln zuverlässig messbar sind. Dadurch ist ein weiterer Parameterraum zur Anpassung von Sensibilitäten an spezifische Gase eröffnet. Die Nanopartikel sind insbesondere auf die Matrix der Sensorstruktur aufdruckbar. Die unterschiedlichen leitenden Elemente der Sensorstruktur bzw. der Matrix sind bevorzugt mit verschiedenen bzw. unterschiedlichen Nanopartikeln dotiert. Es können jedoch auch gleiche Nanopartikel vorgesehen sein.

Es ist von Vorteil, wenn die in der Matrix angeordneten leitenden Elemente zumindest teilweise aus Zinnoxid und/oder Zinkoxid und zumindest teilweise aus Kupferoxid gebildet sind, wobei beispielsweise das Zinnoxid und/oder Zinkoxid n-dotiert und das Kupferoxid p-dotiert ist. Es werden dadurch Elektronendonatoren in das Zinnoxid und/oder Zinkoxid und Elektronenakzeptoren in das Kupferoxid implantiert. Eine intrinsische Dotierung des Metalloxids kann durch zusätzliches Dotieren umgekehrt werden. Die leitenden Elemente können auch zumindest teilweise aus einem anderen Metalloxid gebildet sein. Die in der Matrix angeordneten leitenden Elemente umfassen beispielsweise Metalloxidfilme aus unterschiedlichen Metalloxiden. Alternativ dazu können die leitenden Elemente Kontaktelemente aus unterschiedlichen Metallen und/oder Metalloxiden umfassen, welche über beispielsweise Nanofasern miteinander verbunden sind. Ferner können die leitenden Elemente auch Nanofasern umfassen, welche gitterförmig zueinander angeordnet sind. Die Matrix aus dotiertem Zinnoxid und/oder Zinkoxid und dotiertem Kupferoxid bzw. die in der Matrix angeordneten leitenden Elemente sind miteinander verschaltbar, wodurch eine Diode gebildet wird. Dadurch ergibt sich neben der Möglichkeit einzelne Sensoren miteinander zu koppeln und gegeneinander zu verschalten auch der Effekt, dass Strom in eine Richtung ungehindert durchkommt und in der anderen Richtung gesperrt ist.

Zweckmäßigerweise ist bei der Sensoranordnung, wobei die Matrix Metalloxidfilme umfasst, bei einem Kreuzungspunkt zwischen etwa senkrecht zueinander angeordneten Metallfilmen ein Übergang zwischen Bereichen mit unterschiedlicher Dotierung ausgebildet. Es kann ein p-n Übergang oder ein n-p Übergang ausgebildet sein. Durch eine Matrixverschaltung ist auch ein n-p-n Übergang oder ein p-n-p Übergang erzeugbar, wodurch ein Transistor gebildet ist. Es sind also verschiedene Übergänge zwischen Bereichen mit unterschiedlicher Dotierung freischaltbar. Die Metallfilme sind als Stränge mit unterschiedlichen Metalloxiden oder bündelförmigen Nanofasern mit unterschiedlichen Metalloxiden ausgebildet bzw. in Metalloxidfilmen umgewandelt, wobei die Metalloxidfilme unterschiedlich dotiert sind.

Vorteilhaft ist es weiter, wenn die Sensoranordnung flexibel konfigurierbar ist. Die Sensoranordnung bildet mehrere voneinander beabstandete Sensoren aus, wobei mit Vorteil jeder Sensor auf ein unterschiedliches Gas sensibilisiert ist bzw. reagiert. Abhängig davon, wie die einzelnen Kontakte abgegriffen werden, sind unterschiedliche Gase erkennbar und messbar. Eine flexibel und individuell konfigurierbare Sensoranordnung ist beispielsweise als Smartsensor einsetzbar. Dieser ist insbesondere in der Gebäudesteuerung oder bei einer Steuerung einer Klimaanlage verwendbar.

Um die Sensoranordnung zusätzlich zur Messung bestimmter Gase sensibilisieren zu können, ist es vorteilhaft, wenn die Sensoranordnung eine Heizeinrichtung und/oder eine Kühleinrichtung umfasst, um die Sensoranordnung auf eine definierte Temperatur zu bringen oder definierten Temperaturzyklen zu unterwerfen.

Eine Verwendung einer erfindungsgemäßen Sensoranordnung erfolgt mit Vorteil zur gleichzeitigen Bestimmung von unterschiedlichen Gasen.

Das weitere Ziel wird erreicht, wenn ein Verfahren der eingangs genannten Art folgende Schritte umfasst:
- Bereitstellen eines Substrates,
- Aufbringen einer Sensorstruktur aus unterschiedlichen Metalloxiden umfassend in einer Matrix angeordnete leitende Elemente und eine Vielzahl von Kontakten auf das Substrat und
- Funktionalisieren von in der Sensorstruktur ausgebildeten Sensoren.

Ein damit erzielter Vorteil ist insbesondere darin zu sehen, dass die Kombination des Aufbringens der Sensorstruktur mit in einer Matrix angeordneten leitenden Elementen und mehreren Kontakten und des Funktionalisierens der Sensorstruktur, eine Herstellung einer Sensoranordnung zur isolierten Messung von mehr als zwei Gasen in einem Gasgemisch erlaubt. Bei einer durch das erfindungsgemäße Verfahren hergestellten Sensoranordnung können einzelne, voneinander beabstandete Sensoren in einer Matrix derart miteinander gekoppelt werden, dass diese beliebig extern miteinander angesteuert und verschaltet werden können. Insbesondere können verschiedene Arten von Sensoren miteinander gekoppelt und gegeneinander verschaltet werden, sodass in einem Gasgemisch mehr als zwei unterschiedliche Gase erkannt und individuell gemessen werden können.

Die Sensorstruktur bzw. die Matrix wird aus unterschiedlichen Metalloxiden gebildet, beispielsweise aus Kupferoxid und/oder Zinkoxid und/oder Zinnoxid. Insbesondere wird die Sensorstruktur aus gitterförmig angeordneten Strängen aus unterschiedlichen Metalloxiden gebildet. Hierbei ist es günstig, wenn die Stränge etwa senkrecht zueinander angeordnet werden. Weiter ist es vorteilhaft, wenn die Stränge aus unterschiedlichen Metalloxiden hergestellt werden. Die Stränge können mit Vorteil auch aus Nanofasern gebildet sein. Darüber hinaus können auch Kontaktelemente matrixförmig auf dem Substrat angeordnet werden. Dabei ist es günstig, wenn diese aus unterschiedlichen Metalloxiden ausgebildet und gitterförmig miteinander verbunden werden.

Es kann ferner von Vorteil sein, wenn die Sensorstruktur aus n-dotiertem Zinnoxid und/oder Zinkoxid und aus p-dotiertem Kupferoxid gebildet wird. Hierfür werden Elektronendonatoren in das Zinnoxid und/oder Zinkoxid und Elektronenakzeptoren in das Kupferoxid implantiert. Die Matrix der Sensorstruktur umfasst beispielsweise Metalloxidfilme aus unterschiedlichen Metalloxiden. Die Matrix bzw. Elemente aus beispielsweise dotiertem Zinkoxid und/oder Zinnoxid und dotiertem Kupferoxid der Matrix können miteinander bzw. untereinander verschaltet werden, wodurch eine Diode gebildet wird. Dadurch ergibt sich neben der Möglichkeit einzelne Sensoren miteinander zu koppeln und gegeneinander zu verschalten auch der Effekt, dass Strom in eine Richtung ungehindert durchkommt und in der anderen Richtung gesperrt wird.

Es ist von Vorteil, wenn die Sensorstruktur mit Nanopartikeln funktionalisiert wird. Eine Funktionalisierung kann durch Sensibilisierung erfolgen, wobei die Nanopartikel insbesondere auf die Matrix der Sensorstruktur aufgebracht werden, insbesondere aufgedruckt, beispielsweise mit einem Tintenstrahldrucker. Die Sensorstruktur ist dadurch unterschiedlich funktionalisierbar. Durch die Nanopartikel werden den ausgebildeten Sensoren unterschiedliche Eigenschaften und/oder Sensibilitäten zugeordnet, wodurch in weiterer Folge zwei oder mehr Gase isoliert voneinander zuverlässig gemessen werden können. Somit wird ein weiterer Parameterraum zur Anpassung von Sensibilitäten an spezifische Gase eröffnet. Unterschiedliche Elemente der Sensorstruktur bzw. der Matrix werden bevorzugt mit verschiedenen Nanopartikeln sensibilisiert bzw. dotiert. Es kann jedoch auch nur eine einzige Art von Nanopartikeln zur Sensibilisierung der Sensorstruktur verwendet werden.

Weiter kann eine Funktionalisierung durch klassische Dotierung erfolgen. Neben einer klassischen Dotierung können alternativ oder zusätzlich auch andere Fremdatome oder Nanopartikel, beispielsweise Edelmetalle wie Palladium, Platin etc. in Elemente der Sensorstruktur eingebettet oder aufgebracht werden, um diese für unterschiedliche Gase empfindlich zu machen.

Vorteilhaft ist es, wenn zwischen in der Matrix angeordneten Kontaktelementen, insbesondere metallischen und/oder metalloxidischen Kontaktelementen, Nanofasern auf das Substrat oxidiert werden. Die Kontaktelemente wachsen entweder direkt auf dem Substrat oder werden auf das Substrat aufgebracht, beispielsweise durch Aufdampfen. Die Nanofasern werden etwa senkrecht zueinander zwischen den Kontaktelementen thermisch auf das Substrat oxidiert. Durch die mit den Nanofasern verbundenen Kontaktelemente wird ein Gitter ausgebildet, wobei Bündel von Nanofasern vorgesehen sind. Die Kontaktelemente sind mit Vorteil etwa rechteckig, insbesondere quadratisch, ausgebildet und matrixförmig auf einem Substrat angeordnet. Die äußeren Kontaktelemente der dadurch gebildeten Matrix sind jeweils mit einem Kontakt verbunden. Alternativ dazu können die Nanofasern auch auf das Substrat gedruckt werden.

Es kann günstig sein, wenn Metalloxidfilme auf das Substrat aufgedampft werden. Die Metalloxidfilme sind insbesondere streifenförmig ausgebildet und werden gitterförmig bzw. matrixförmig auf das Substrat aufgedampft. An jeweiligen Enden der streifenförmigen Metalloxidfilme werden Kontakte angeordnet, über die unterschiedlich ausgebildete Sensoren angesteuert werden können. Sensoren werden an und/oder zwischen Kreuzungspunkten der leitenden Elemente ausgebildet, wobei die Kreuzungspunkte mit Abstand zueinander auf der Sensorstruktur angeordnet sind. Es ist zweckmäßig, wenn die leitenden Elemente einer Reihe bzw. Spalte zumindest teilweise aus unterschiedlichen Metalloxiden gebildet werden. Es kann auch zweckmäßig sein, wenn die Metalloxidfilme als Bündel von Nanofasern ausgebildet sind.

Weitere Merkmale, Vorteile und Wirkungen ergeben sich aus den nachfolgend dargestellten Ausführungsbeispielen. In den Zeichnungen, auf welche dabei Bezug genommen wird, zeigen:
Fig. 1a eine Sensoranordnung;
Fig. 1b eine schematische Darstellung einer Verschaltung zweier Kontakte;
Fig. 2 eine erfindungsgemäße Sensoranordnung;
Fig. 3 eine weitere erfindungsgemäße Sensoranordnung;
Fig. 4 eine weitere erfindungsgemäße Sensoranordnung;
Fig. 5 ein Bild von Kontaktelementen;
Fig. 6 ein weiteres Bild von Kontaktelementen;
Fig. 7 ein weiteres Bild von Kontaktelementen;
Fig. 8 ein weiteres Bild von Kontaktelementen;
Fig. 9 ein Bild einer zweidimensionalen Verbindung von Kontaktelementen.

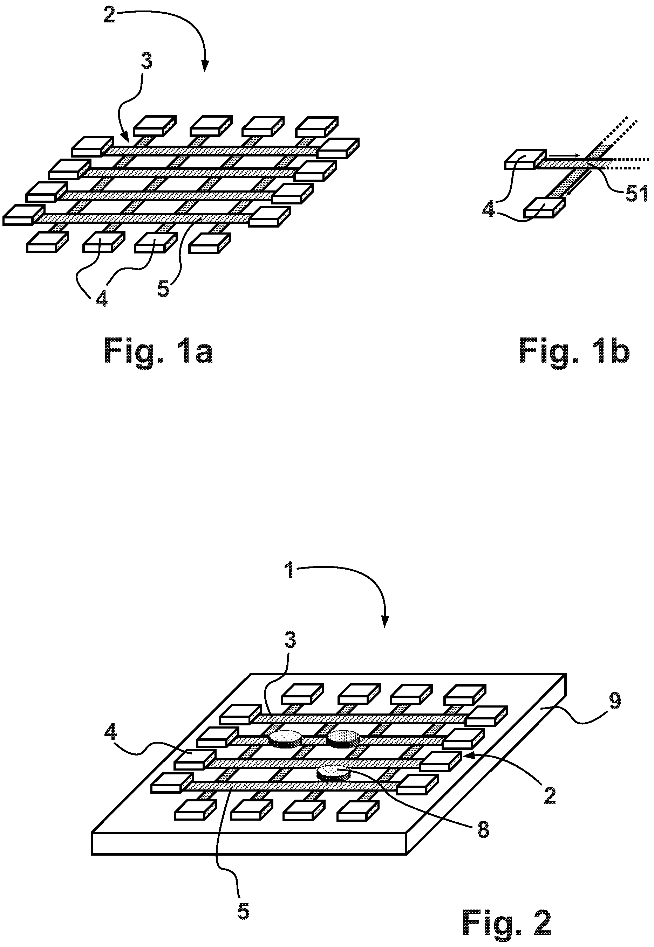

Fig. 1a zeigt eine Sensorstruktur 2 für eine erfindungsgemäße Sensoranordnung 1. Die Sensorstruktur 2 umfasst in einer Matrix 3 angeordnete leitende Elemente und mehrere Kontakte 4, welche beispielsweise aus Silber oder Titan ausgebildet sind. In Fig. 1a umfasst die Matrix 3 Metalloxidfilme 5 bzw. ist die Matrix 3 aus Metalloxidfilmen 5 gebildet. Die Metalloxidfilme 5 sind gitterförmig angeordnet, wobei an den zwei Enden eines jeden Metalloxidfilmes 5 die Kontakte 4 vorgesehen bzw. mit den als Metalloxidfilme 5 ausgebildeten leitenden Elementen verbunden sind.

Fig. 1b zeigt ein Beispiel einer Verschaltung zweier Kontakte 4 mittels eines n-p Überganges an einem Knotenpunkt 51.

Die Sensorstruktur 2 bzw. die leitenden Elemente sind mit unterschiedlichen Metalloxiden gebildet, z. B. aus Kupferoxid und/oder Zinkoxid und/oder Zinnoxid. Die Kontakte 4 sind über die Metalloxidfilme 5 miteinander verbunden, wobei die Kontakte 4 beliebig miteinander koppelbar und verschaltbar sind, wodurch in weiterer Folge mehr als zwei Gase messbar sind.

Fig. 2 zeigt eine erfindungsgemäße Sensoranordnung 1. Diese umfasst eine Sensorstruktur 2 mit in einer Matrix 3 angeordneten leitenden Elementen und einer Vielzahl von Kontakten 4, welche auf einem Substrat 9 angeordnet ist. Die Sensorstruktur 2 entspricht der Sensorstruktur 2 gemäß Fig. 1a. Die leitenden Elemente sind wiederum aus gitterförmig angeordneten Metalloxidfilmen 5 gebildet. Die Metalloxidfilme 5 sind darüber hinaus mit Nanopartikeln 8 sensibilisiert.

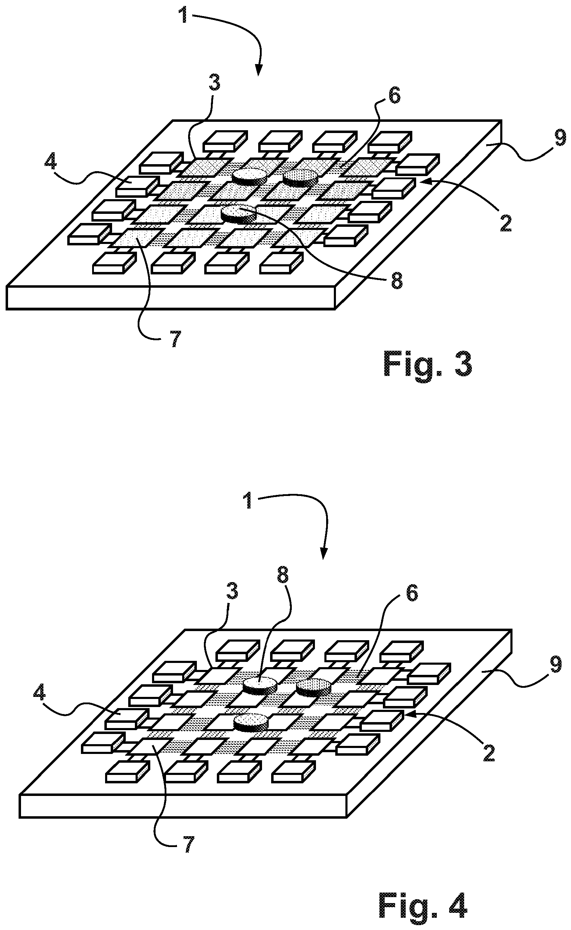

In Fig. 3 ist eine weitere erfindungsgemäße Sensoranordnung 1 mit einer Sensorstruktur 2, umfassend aus Metalloxiden gebildete und in einer Matrix 3 angeordnete leitende Elemente und Kontakte 4, gezeigt. Die Matrix 3 umfasst Kontaktelemente 7 aus unterschiedlichen Metallfilmen und/oder Metalloxidfilmen 5, welche über metalloxidische Nanofasern 6 miteinander verbunden sind. Die Nanofasern 6 sind mit Nanopartikeln 8 sensibilisiert bzw. funktionalisiert. Die Kontaktelemente 7 werden unmittelbar auf dem Substrat 9 angeordnet, wohingegen die Nanofasern 6 auf das Substrat 9 bzw. einen Wafer oxidiert werden.

Eine weitere erfindungsgemäße Sensoranordnung 1 ist in Fig. 4 gezeigt. Diese Sensoranordnung 1 entspricht im Wesentlichen der Sensoranordnung 1 gemäß Fig. 3. Hier werden die Kontaktelemente 7 auf das Substrat 9 aufgebracht und die Nanofasern 6 zwischen den Kontaktelementen 7 diese verbindend auf das Substrat 9 gedruckt.

In allen Ausführungsvarianten einer erfindungsgemäßen Sensoranordnung 1 sind die leitenden Elemente der Matrix 3 zumindest teilweise aus unterschiedlichen Metalloxiden gebildet, beispielsweise aus Kupferoxid und/oder Zinkoxid und/oder Zinnoxid. Zusätzlich können die leitenden Elemente der Sensorstruktur 2 n-dotiert und/oder p-dotiert sein.

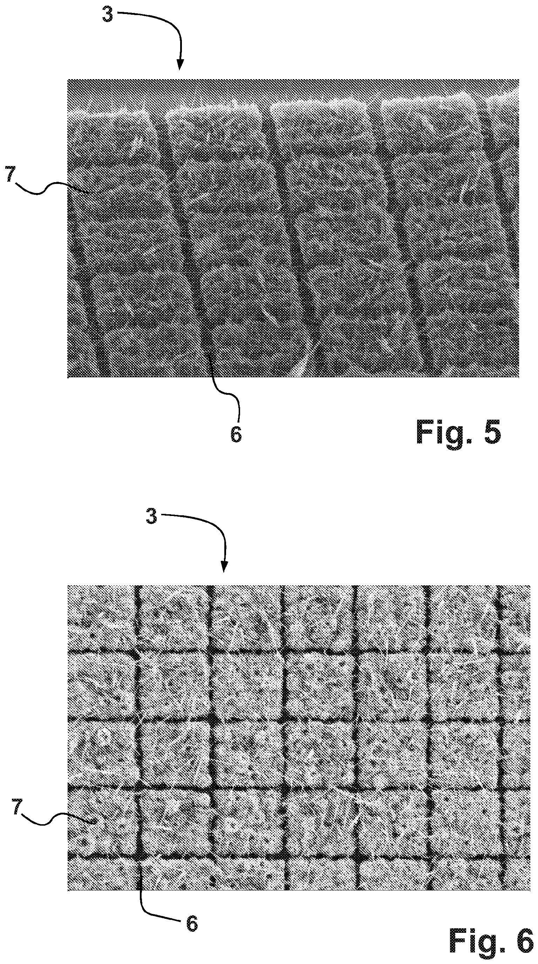

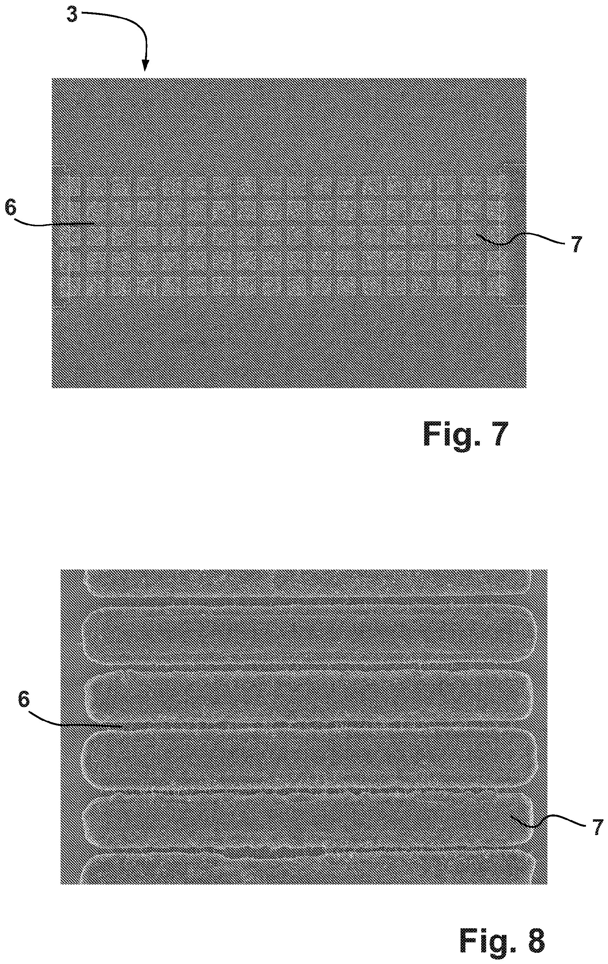

Fig. 5, 6, 7 und 8 zeigen jeweils ein mit einem Rasterelektronenmikroskop aufgenommenes Bild von Kontaktelementen 7 mit Nanofasern 6. Die Nanofasern 6 werden bei einer Herstellung einer solchen Matrix 3 zwischen den Kontaktelementen 7 thermisch oxidiert, wohingegen die Kontaktelemente 7 direkt auf einem Substrat 9 aufgebracht werden, beispielsweise durch Aufdampfen, Aufdrucken, Sputtern oder elektrolytisch. In Fig. 5 bis 7 ist eine Matrixverschaltung der Kontaktelemente 7 über die Nanofasern 6 bzw. eine Matrix 3 abgebildet, wohingegen Fig. 8 eine reine Verbindung von Kontaktelementen 7 über Nanofasern 6 zeigt. Diese Aufnahmen sind allerdings nur exemplarisch, da die Nanofasern 6 hierbei jeweils nur aus einem Metalloxid gebildet sind (Kupferoxid in Fig. 5, 6 und 7; Zinkoxid in Fig. 8). Bei einer erfindungsgemäßen Sensoranordnung 1 ist die Matrix 3 aus unterschiedlichen Metalloxiden gebildet, um mehr als zwei Gase eines Gasgemisches bestimmen und messen zu können.

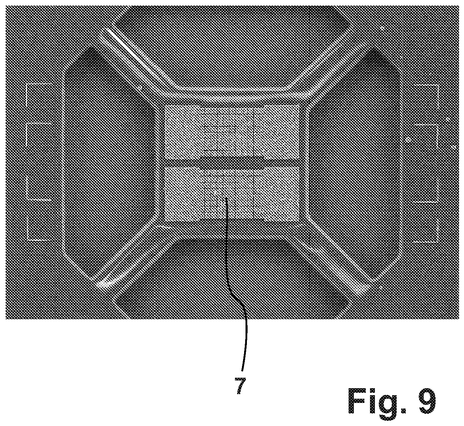

In Fig. 9 ist ein mit einem Rasterelektronenmikroskop aufgenommenes Bild einer zweidimensionalen Verbindung von Kontaktelementen 7 gezeigt. Ein Träger der Sensoranordnung 1 kann beispielsweise eine Mikro-Heizplatte sein, mit der die Sensoranordnung 1 auf eine definierte Temperatur gebracht oder definierten Temperaturzyklen unterworfen werden kann.

Bei einem erfindungsgemäßen Verfahren zur Herstellung einer Sensoranordnung 1 zur Bestimmung von mehreren Gasen wird ein Substrat 9 bereitgestellt, auf welches eine Sensorstruktur 2, umfassend eine Matrix 3 aus leitenden Elementen und eine Vielzahl von Kontakten 4, aufgebracht wird. In einem nächsten Schritt wird die Sensorstruktur 2 insbesondere mit Nanopartikeln 8 auf mehrere Gase sensibilisiert.

Die Arbeit, welche zu dieser Erfindung geführt hat, wurde gefördert von der Europäischen Union, Seventh Framework Programme (*FP7*/*2007*-*2013*) unter dem *grant agreement* Nr. 611887.

## Patentansprüche

1. Sensoranordnung (1) zur Bestimmung und gegebenenfalls Messung einer Konzentration von mehreren Gasen, umfassend eine Sensorstruktur (2) mit in einer Matrix (3) angeordneten leitenden Elementen und einer Vielzahl von Kontakten (4), wobei die leitenden Elemente mit den Kontakten (4) verbunden sind, **dadurch gekennzeichnet, dass** die Sensorstruktur (2) eine Matrix aus gitterförmig angeordneten Strängen aus leitenden Elementen aus unterschiedlichen Metalloxiden umfasst, wobei die Stränge an Kreuzungspunkten übereinander laufen, wobei die leitenden Elemente derart angeordnet sind, dass die Sensoranordnung (1) mehrere miteinander koppelbare Sensoren ausbildet, welche individuell über die Kontakte (4) ansteuerbar und untereinander verschaltbar sind, um in einem Gasgemisch mehr als zwei Gase isoliert voneinander zu messen, wobei die Sensoren beabstandet voneinander an Berührungspunkten zwischen zwei oder mehr leitenden Elementen angeordnet sind.

2. Sensoranordnung (1) nach Anspruch 1, **dadurch gekennzeichnet, dass** die Matrix (3) Metallfilme und/oder Metalloxidfilme (5) umfasst.

3. Sensoranordnung (1) nach Anspruch 1 oder 2, **dadurch gekennzeichnet, dass** die Sensorstruktur (2) Nanofasern (6) umfasst, wobei die Nanofasern (6) auf bzw. zwischen leitenden Elementen der Metallmatrix (3) angeordnet sind, wobei die Nanofasern (6) über einen Transferprozess auf ein Substrat übertragen werden..

4. Sensoranordnung (1) nach Anspruch 3, **dadurch gekennzeichnet, dass** Kontaktelemente (7), insbesondere metallische und/oder metalloxidische Kontaktelemente (7), vorgesehen sind, wobei die Nanofasern (6) zwischen den Kontaktelementen (7) angeordnet sind.

5. Sensoranordnung (1) nach Anspruch 3 oder 4, **dadurch gekennzeichnet, dass** die Nanofasern (6) dotiert sind.

6. Sensoranordnung (1) nach einem der Ansprüche 1 bis 5, **dadurch gekennzeichnet, dass** die Sensorstruktur (2) Nanopartikel (8) umfasst, um Sensoren unterschiedlich zu sensibilisieren.

7. Sensoranordnung (1) nach einem der Ansprüche 1 bis 6, **dadurch gekennzeichnet, dass** die in der Matrix (3) angeordneten leitenden Elemente zumindest teilweise aus Zinnoxid und/oder Zinkoxid und zumindest teilweise aus Kupferoxid gebildet sind, wobei beispielsweise das Zinnoxid und/oder Zinkoxid n-dotiert und das Kupferoxid p-dotiert ist.

8. Sensoranordnung (1) nach einem der Ansprüche 1 bis 7, **dadurch gekennzeichnet, dass** die Matrix (3) Metallfilme und/oder Metalloxidfilme (5) umfasst und bei einem Kreuzungspunkt zwischen etwa senkrecht zueinander angeordneten Metallfilmen und/oder Metalloxidfilmen (5) ein Übergang zwischen Bereichen mit unterschiedlicher Dotierung ausgebildet ist.

9. Sensoranordnung (1) nach einem der Ansprüche 1 bis 8, **dadurch gekennzeichnet, dass** die Sensoranordnung (1) mehrere voneinander beabstandete Sensoren ausbildet, wobei jeder Sensor auf ein unterschiedliches Gas sensibilisiert ist bzw. reagiert, wodurch die Sensoranordnung (1) flexibel konfigurierbar ist.

10. Sensoranordnung (1) nach einem der Ansprüche 1 bis 9, **dadurch gekennzeichnet, dass** die Sensoranordnung (1) eine Heizeinrichtung und/oder eine Kühleinrichtung umfasst, um die Sensoranordnung (1) auf eine definierte Temperatur zu bringen oder definierten Temperaturzyklen zu unterwerfen.

11. Verwendung einer Sensoranordnung (1) nach einem der Ansprüche 1 bis 10 zur gleichzeitigen Bestimmung von unterschiedlichen Gasen.

12. Verfahren zur Herstellung einer Sensoranordnung (1) zur Bestimmung und gegebenenfalls Messung einer Konzentration von mehreren Gasen, insbesondere einer Sensoranordnung (1) nach einem der Ansprüche 1 bis 11, umfassend folgende Schritte:
- Bereitstellen eines Substrates (9),
- Aufbringen einer Sensorstruktur (2) aus unterschiedlichen Metalloxiden umfassend in einer Matrix (3) gitterförmig angeordnete leitende Elemente und eine Vielzahl von Kontakten (4) auf das Substrat (9),
- Funktionalisieren von in der Sensorstruktur (2) ausgebildeten Sensoren und
- Koppeln von mehreren voneinander beabstandeten und an Berührungspunkten zwischen zwei oder mehr leitenden Elementen angeordneten Sensoren, sodass diese individuell über die Kontakte (4) angesteuert und untereinander verschaltet werden können.

13. Verfahren nach Anspruch 12, **dadurch gekennzeichnet, dass** die Sensorstruktur (2) mit Nanopartikeln (8) funktionalisiert wird.

14. Verfahren nach Anspruch 12 oder 13, **dadurch gekennzeichnet, dass** zwischen in der Matrix (3) angeordneten Kontaktelementen (7), insbesondere metallischen und/oder metalloxidischen Kontaktelementen (7), Nanofasern (6) auf das Substrat (9) oxidiert werden.

15. Verfahren nach einem der Ansprüche 12 bis 14, **dadurch gekennzeichnet, dass** Metallfilme und/oder Metalloxidfilme (5) auf das Substrat (9) aufgebracht werden.

## Claims

1. A sensor arrangement (1) for determining and optionally measuring a concentration of multiple gases, comprising a sensor structure (2) with conductive elements arranged in a matrix (3) and a plurality of contacts (4), wherein the conductive elements are connected to the contacts (4), **characterized in that** the sensor structure (2) comprises a matrix of strands of conductive elements, which are arranged in a grid-shaped manner and consist of different metal oxides, wherein the strands extend on top of one another at intersecting points, wherein the conductive elements are arranged in such a way that the sensor arrangement (1) forms multiple sensors, which can be coupled to one another and individually activated and interconnected by means of the contacts (4) in order to measure more than two gases in a gas mixture in isolation from one another, and wherein the sensors are arranged at a distance from one another at contact points between two or more conductive elements.

2. The sensor arrangement (1) according to claim 1, **characterized in that** the matrix (3) comprises metal films and/or metal oxide films (5).

3. The sensor arrangement (1) according to claim 1 or 2, **characterized in that** the sensor structure (2) comprises nanofibers (6), wherein the nanofibers (6) are respectively arranged on or between conductive elements of the metal matrix (3), and wherein the nanofibers (6) are transferred onto a substrate by means of a transfer process.

4. The sensor arrangement (1) according to claim 3, **characterized in that** contact elements (7), particularly metallic and/or metal-oxidic contact elements (7), are provided, wherein the nanofibers (6) are arranged between the contact elements (7).

5. The sensor arrangement (1) according to claim 3 or 4, **characterized in that** the nanofibers (6) are doped.

6. The sensor arrangement (1) according to one of claims 1 to 5, **characterized in that** the sensor structure (2) comprises nanoparticles (8) in order to sensitize sensors differently.

7. The sensor arrangement (1) according to one of claims 1 to 6, **characterized in that** the conductive elements arranged in the matrix (3) are at least partially made of tin oxide and/or zinc oxide and at least partially of copper oxide, and wherein, for example, the tin oxide and/or zinc oxide is n-doped and the copper oxide is p-doped.

8. The sensor arrangement (1) according to one of claims 1 to 7, **characterized in that** the matrix (3) comprises metal films and/or metal oxide films (5) and a transition between regions with different doping is formed at an intersecting point between metal films and/or metal oxide films (5) that are arranged approximately perpendicular to one another.

9. The sensor arrangement (1) according to one of claims 1 to 8, **characterized in that** the sensor arrangement (1) forms multiple sensors that are spaced apart from one another, wherein each sensor respectively is sensitized or reacts to a different gas such that the sensor arrangement (1) can be flexibly configured.

10. The sensor arrangement (1) according to one of claims 1 to 9, **characterized in that** the sensor arrangement (1) comprises a heating device and/or a cooling device in order to bring the sensor arrangement (1) to a defined temperature or to subject the sensor arrangement to defined temperature cycles.

11. The use of the sensor arrangement (1) according to one of claims 1 to 10 for simultaneously determining different gases.

12. A method for producing a sensor arrangement (1) for determining and optionally measuring a concentration of multiple gases, particularly a sensor arrangement (1) according to one of claims 1 to 11, comprising the following steps:
- providing a substrate (9),
- applying a sensor structure (2) of different metal oxides comprising conductive elements arranged in a matrix (3) in a grid-shaped manner and a plurality of contacts (4) onto the substrate (9),
- functionalizing sensors formed in the sensor structure (2) and
- coupling multiple sensors that are spaced apart from one another and arranged at contact points between two or more conductive elements such that these sensors can be individually activated and interconnected by means of the contacts (4).

13. The method according to claim 12, **characterized in that** the sensor structure (2) is functionalized with nanoparticles (8).

14. The method according to claim 12 or 13, **characterized in that** nanofibers (6) are oxidized onto the substrate (9) between contact elements (7), particularly metallic and/or metal-oxidic contact elements (7), arranged in the matrix (3).

15. The method according to one of claims 12 to 14, **characterized in that** metal films and/or metal oxide films (5) are applied onto the substrate (9).

## Revendications

1. Système de détection (1) pour déterminer et le cas échéant mesurer d'une concentration de plusieurs gaz, comprenant une structure de détection (2) avec des éléments conducteurs disposés dans une matrice (3) et un grand nombre de contacts (4), sachant que les éléments conducteurs sont reliés aux contacts (4), **caractérisé en ce que** la structure de détection (2) comprend une matrice en brins disposés en forme de grille en éléments conducteurs d'oxydes métalliques différents, sachant que les brins passent les uns sur les autres aux points de croisement, sachant que les éléments conducteurs sont disposés de telle manière que le système de détection (1) est constitué de plusieurs capteurs pouvant être couplés les uns aux autres, lesquels peuvent être activés et connectés entre eux individuellement par les contacts (4) pour mesurer isolés les uns des autres plus de deux gaz dans un mélange de gaz, sachant que les capteurs sont disposés à distance les uns des autres à des points de contact entre deux éléments conducteurs ou plus.

2. Système de détection (1) selon la revendication 1, **caractérisé en ce que** la matrice (3) comprend des films métalliques et/ou des films d'oxyde métallique (5).

3. Système de détection (1) selon la revendication 1 ou 2, **caractérisé en ce que** la nanostructure (2) comprend des nanofibres (6), sachant que les nanofibres (6) sont disposées sur ou entre les éléments conducteurs de la matrice métallique (3), sachant que les nanofibres (6) sont transférées par un processus de transfert sur un substrat.

4. Système de détection (1) selon la revendication 3, **caractérisé en ce que** des éléments de contact (7), en particulier des éléments de contact (7) métalliques et/ou d'oxyde métallique, sont prévus, sachant que les nanofibres (6) sont disposées entre les éléments de contact (7).

5. Système de détection (1) selon la revendication 3 ou 4, **caractérisé en ce que** les nanofibres (6) sont dopées.

6. Système de détection (1) selon l'une quelconque des revendications 1 à 5, **caractérisé en ce que** la structure de détection (2) comprend des nanoparticules (8) pour sensibiliser les capteurs de façon différente.

7. Système de détection (1) selon l'une quelconque des revendications 1 à 6, **caractérisé en ce que** les éléments conducteurs disposés dans la matrice (3) sont formés au moins en partie d'oxyde d'étain et/ou d'oxyde de zinc et au moins en partie d'oxyde de cuivre, sachant par exemple que l'oxyde d'étain et/ou l'oxyde de zinc sont dopé n et l'oxyde de cuivre est dopé p.

8. Système de détection (1) selon l'une quelconque des revendications 1 à 7, **caractérisé en ce que** la matrice (3) comprend des films métalliques et/ou des films d'oxyde métallique (5) et qu'un transfert est constitué entre des zones avec un dopage différent à un point de croisement entre les films métalliques et/ou films d'oxyde métallique (5) disposés à peu près perpendiculairement les uns par rapport aux autres.

9. Système de détection (1) selon l'une quelconque des revendications 1 à 8, **caractérisé en ce que** le système de détection (1) est constitué de plusieurs capteurs à distance les uns des autres, sachant que chaque capteur est sensibilisé ou réagit à un gaz différent, le système de détection (1) pouvant être de ce fait configuré de façon souple.

10. Système de détection (1) selon l'une quelconque des revendications 1 à 9, ***caractérisé en ce que*** le système de détection (1) comprend un dispositif de chauffage et/ou un dispositif de refroidissement pour porter le système de détection (1) à une température définie ou le soumettre à des cycles de températures définis.

11. Utilisation d'un système de détection (1) selon l'une quelconque des revendications 1 à 10 pour la détermination simultanée de gaz différents.

12. Procédé de fabrication d'un système de détection (1) pour déterminer et le cas échéant mesurer une concentration de plusieurs gaz, en particulier d'un système de détection (1) selon l'une quelconque des revendications 1 à 11, comprenant les étapes suivantes :
- préparation d'un substrat (9),
- application d'une structure de détection (2) en différents oxydes métalliques comprenant des éléments conducteurs disposés en forme de grille dans une matrice (3) et un grand nombre de contacts (4) sur le substrat (9),
- fonctionnalisation de capteurs constitués dans la structure de détection (2) et
- couplage de plusieurs capteurs disposés à distance les uns des autres et sur des points de contact entre deux éléments conducteurs ou plus de telle sorte que ceux-ci puissent être activés individuellement par les contacts (4) et être connectés entre eux.

13. Procédé selon la revendication 12, **caractérisé en ce que** la structure de détection (2) est fonctionnalisée avec des nanoparticules (8).

14. Procédé selon la revendication 12 ou 13, **caractérisé en ce que** des nanofibres (6) sont oxydées sur le substrat (9) entre des éléments de contacts (7) disposés dans la matrice (3), en particulier des éléments de contacts (7) métalliques et/ou d'oxyde métallique.

15. Procédé selon l'une quelconque des revendications 12 à 14, **caractérisé en ce que** des films métalliques et/ou des films d'oxyde métallique (5) sont appliqués sur le substrat (9).
